(19) Europäisches Patentamt / European Patent Office / Office européen des brevets

(11) **EP 2 559 376 A1**

(12) **EUROPEAN PATENT APPLICATION**

(43) Date of publication:
20.02.2013 Bulletin 2013/08

(51) Int Cl.:
*A61B 5/01* (2006.01)  *A61B 8/00* (2006.01)
*A61N 7/02* (2006.01)  *G01K 11/02* (2006.01)

(21) Application number: 12180879.4

(22) Date of filing: 17.08.2012

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: 18.08.2011 KR 20110082426

(71) Applicant: Samsung Electronics Co., Ltd.
Suwon-si, Gyeonggi-do, 443-742 (KR)

(72) Inventors:
• Choi, Ki-wan
  Gyeonggi-do (KR)
• Kong, Dong-geon
  Gyeonggi-do (KR)
• Seo, Jong-bum
  Seoul (KR)

(74) Representative: D'Halleweyn, Nele Veerle Trees Gertrudis et al
Arnold & Siedsma
Sweelinckplein 1
2517 GK Den Haag (NL)

(54) **Method of generating ultrasound image and ultrasound system using the method**

(57) A method of generating an ultrasound image and an ultrasound system using the method, the method including: obtaining an echo signal from an ultrasound signal irradiated on an organ; calculating an amount of a change in the echo signal with respect to a reference echo signal; determining a temperature of the organ from the amount of the change in the echo signal by using an amount of a change in an attenuation coefficient of the organ that changes according to a temperature change; and generating an ultrasound image including information about the determined temperature.

FIG. 1

EP 2 559 376 A1

**Description**

Example embodiments of the following disclosure relate to methods of generating ultrasound images and ultrasound systems using the methods, and more particularly, to methods of generating ultrasound images and ultrasound systems using the methods, in which the temperature of an organ is determined by using the amount of a change in an attenuation coefficient of an organ.

As medical science has advanced, the loco-regional treatment of tumors has also developed in relation to invasive surgery, such as, laparotomy and minimal-invasive surgery. In addition, as non-invasive surgery techniques have progressed, a gamma knife, a cyber knife, a high intensity focused ultrasound (HIFU) knife, and the like, have emerged. In particular, the HIFU knife is not harmful to the human body and has been widely used as an echo-friendly treatment.

In HIFU treatment using the HIFU knife, HIFU is irradiated on a tumor part in order to cause focal destruction or necrosis of a tumor tissue, thereby removing the tumor.

A method of monitoring whether tumor necrosis occurs due to ultrasound has been studied. When tumor necrosis occurs due to ultrasound treatment, the temperature of an organ with a tumor generally rises. Thus, an ultrasound system may determine whether tumor necrosis occurs due to a change in the temperature of the organ with the tumor. However, when ultrasound signals advance in a region in which the change in the temperature of the organ with the tumor occurs, a back scattering coefficient and an attenuation coefficient of the organ with the tumor are changed according to the temperature of the organ with the tumor. Accordingly, a false image that shows a temperature change even though there is no actual temperature change in a deeper region than the region in which the change in the temperature of the organ with the tumor occurs may appear in an ultrasound image.

Provided are methods of generating an ultrasound image including temperature information for determining tumor necrosis and ultrasound systems using the method.

Also provided are computer readable recording mediums having recorded thereon programs for executing the methods.

Additional aspects will be set forth in part in the description which follows and, in part, will be apparent from the description, or may be learned by practice of the presented embodiments.

According to an aspect of the present invention, a method of generating a ultrasound image, includes: obtaining an echo signal of ultrasound irradiated on an organ; calculating an amount of a change in the echo signal with respect to a reference echo signal; determining a temperature of the organ from the amount of the change in the echo signal by using an amount of a change in an attenuation coefficient of a predetermined region of the organ that changes according to a temperature change; and generating an ultrasound image including information about the temperature.

When the organ is at a reference temperature, the reference echo signal may be an echo signal of ultrasound irradiated on the organ.

The amount of the change in the attenuation coefficient of the organ may be determined from an amount of a change in a former echo signal that is an amount of a change in an echo signal at a former region than the predetermined region of the organ in an ultrasound probe that irradiates ultrasound on the organ.

The amount of the change in the attenuation coefficient of the organ may be proportional to a standard deviation with respect to the amount of the change in the former echo signal.

A degree of proportion between the standard deviation between the amount of the change in the attenuation coefficient of the organ and the amount of the change in the former echo signal may depend on a type of the organ.

The determining of the temperature of the organ may include: calculating an amount of a change in a back scattering coefficient of the organ from the amount of the change in the attenuation coefficient of the organ and the amount of the change in the echo signal; and determining the temperature of the organ from the back scattering coefficient.

The temperature of the organ may be determined by reading the temperature of the organ that is mapped to the amount of the change in the back scattering coefficient of the organ, from a temperature database in which the amount of the change in the back scattering coefficient of the organ and the temperature of the organ are mapped to each other.

The echo signal of ultrasound may be a signal in which ultrasound is back scattered by the organ and received by the ultrasound probe that irradiates ultrasound on the organ.

The amount of the change in the attenuation coefficient of the organ may depend on a type of the organ.

The ultrasound image may include brightness corresponding to the temperature of the organ.

The method may further include: irradiating ultrasound on the organ; and receiving the echo signal from the organ.

According to another aspect of the present invention, an ultrasound system includes: a ultrasound data obtaining unit for irradiating ultrasound on an organ and for receiving an echo signal of ultrasound from the organ; and a processor for calculating an amount of a change in the echo signal with respect to a reference echo signal and determining a temperature of the organ from the amount of the change in the echo signal by using an amount of a change in an attenuation coefficient of the organ that changes according to a temperature change.

When the organ is at a reference temperature, the reference echo signal may be an echo signal of ultrasound irradiated on the organ.

The amount of the change in the attenuation coefficient of the organ may be determined from an amount of a change in a former echo signal that is an amount of a change in an echo signal at a former region than the predetermined region of the organ in an ultrasound probe that irradiates ultrasound on the organ.

The amount of the change in the attenuation coefficient of the organ may be proportional to a standard deviation with respect to the amount of the change in the former echo signal.

A degree of proportion between the standard deviation between the amount of the change in the attenuation coefficient of the organ and the amount of the change in the former echo signal may depend on a type of the organ.

The processor may calculate an amount of a change in a back scattering coefficient of the organ from the amount of the change in the attenuation coefficient of the organ and the amount of the change in the echo signal and may determine the temperature of the organ from the back scattering coefficient.

The echo signal of ultrasound may be a signal back scattered by the organ and is received by an ultrasound probe that irradiates ultrasound on the organ.

The ultrasound system may further include a display unit for displaying an ultrasound image including information regarding the temperature of the organ.

The ultrasound image may include brightness corresponding to the temperature of the organ.

According to another aspect of the present disclosure, a method of generating an ultrasound image is provided, including: receiving an echo signal of ultrasound; calculating an amount of change in the received echo signal with respect to a reference echo signal; determining a temperature of a predetermined region, based on the amount of change in the received echo signal; and generating the ultrasound image, including information on the determined temperature of the predetermined region.

## BRIEF DESCRIPTION OF THE DRAWINGS

The patent or application file contains at least one drawing executed in color. Copies of this patent or patent application publication with color drawing(s) will be provided by the Office upon request and payment of the necessary fee. These and/or other aspects will become apparent and more readily appreciated from the following description of the embodiments, taken in conjunction with the accompanying drawings of which:

FIG. 1 is a block diagram of a structure of an ultrasound system, according to an example embodiment;
FIG. 2 is a block diagram of a structure of an ultrasound data obtaining unit of the ultrasound system illustrated in FIG. 1;
FIG. 3 is a block diagram of a structure of a processor of the ultrasound system illustrated in FIG. 1;
FIG. 4 is a flowchart illustrating an operation of determining a temperature of an organ from the amount of a change in an echo signal, according to an example embodiment; and
FIG. 5, part (a) and part (b) are an ultrasound image including temperature information that is generated, according to the related art, and an ultrasound image including temperature information that is generated, according to an example embodiment of the present disclosure, respectively.

## DETAILED DESCRIPTION

Reference will now be made in detail to embodiments, examples of which are illustrated in the accompanying drawings, wherein like reference numerals refer to the like elements throughout. In this regard, the present embodiments may have different forms and should not be construed as being limited to the descriptions set forth herein. Accordingly, the embodiments are merely described below, by referring to the figures, to explain aspects of the present description

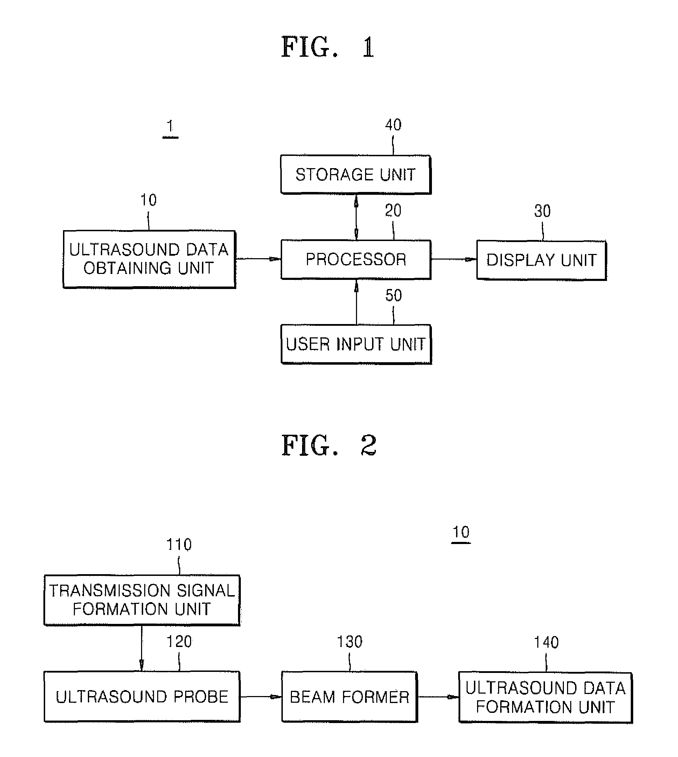

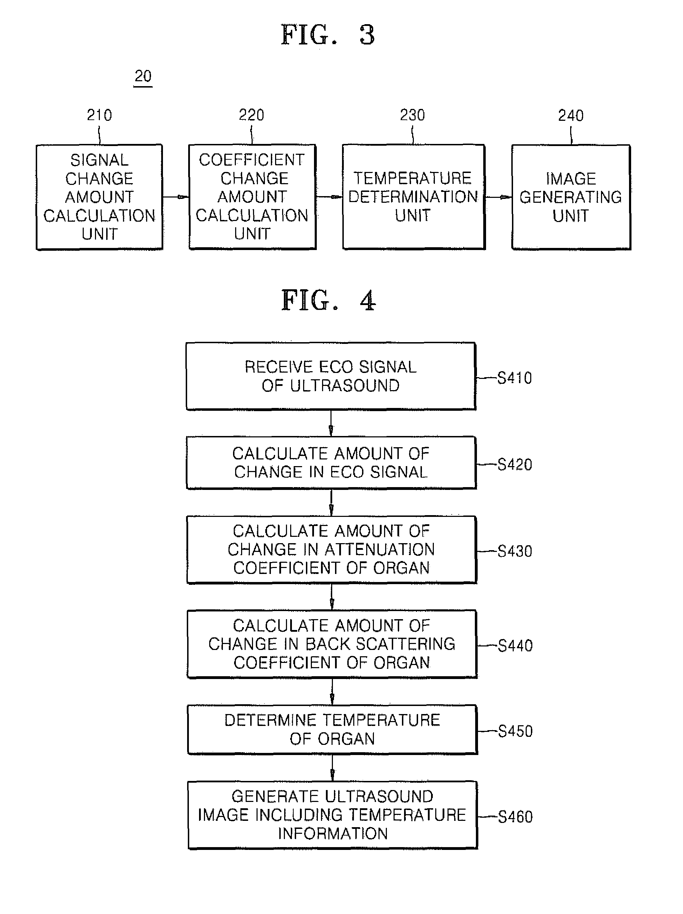

FIG. 1 is a block diagram of an ultrasound system 1, according to an example embodiment. FIG. 2 is a block diagram of an ultrasound data obtaining unit 10 of the ultrasound system 1, as illustrated in FIG. 1. FIG. 3 is a block diagram of a processor 20 of the ultrasound system 1, as illustrated in FIG. 1. The ultrasound system 1 is used for irradiating ultrasound to a tumor that occurs in a patient's body and for generating ultrasound images of the organ with the tumor for diagnosis purposes and for determining whether the treatment has been successful or not. As illustrated in FIG. 1, the ultrasound system 1 includes the ultrasound data obtaining unit 10, the processor 20, and a display unit 30. The ultrasound system 1 may further include a user input unit 50 and a storage unit 40.

The ultrasound data obtaining unit 10 may obtain ultrasound data by transmitting an ultrasound signal to an organ and receiving a reflected ultrasound signal, i.e., an echo signal, from the organ. According to an example embodiment of the present disclosure, the ultrasound data obtaining unit 10 may obtain an intensity of the echo signal.

Referring to FIG. 2, the ultrasound data obtaining unit 10 may include a transmission signal formation unit 110, an ultrasound probe 120 including a plurality of transducer elements (not shown), a beam former 130, and an ultrasound data formation unit 140.

The transmission signal formation unit 110 may form a transmission signal in consideration of positions and focusing points of the plurality of transducer elements (not shown). In the current embodiment, an ultrasound image may include a B-mode (brightness mode) image, a D-mode (doppler mode) image, a C-mode (colordoppler mode) image, an elastic image, a three-dimensional (3D) ultrasound image, or the like. As the transmission signal formation unit 110 sequentially and repeatedly forms transmission signals, a plurality of transmission signals may be formed.

When the transmission signal is provided to the ultrasound probe 120 by the transmission signal formation unit 110, the ultrasound probe 120 may transform the transmission signal into an ultrasound signal, transmit the ultrasound signal to the organ, and receive the reflected ultrasound echo signal from the organ, thereby forming a received signal. The received signal may be an analog signal. The ultrasound probe 120 may transmit and receive the ultrasound signal sequentially and repeatedly according to the transmission signal that is sequentially provided by the transmission signal formation unit 110, thereby forming a plurality of received signals. The ultrasound probe 120 may include a 3D mechanical probe, a two-dimensional (2D) array probe, or the like.

The beam former 130 may convert the received signal provided by the ultrasound probe 120 from analog to digital. Furthermore, the beam former 130 may receive and focus the digital-converted received signal, so as to form a focused signal in consideration of the positions and focusing points of the transducer elements (not shown) of the ultrasound probe 120.

The ultrasound data formation unit 140 may form ultrasound data by using the focused signal provided by the beam former 130. The ultrasound data formation unit 140 may perform a variety of signal processing for forming the ultrasound data, for example, gain control, filtering, etc., on the focused signal, however, the signal processing is not limited thereto.

The processor 20 may obtain information regarding a distance between the ultrasound probe 120 and the organ that generates the echo signal according to the time difference between the time when the ultrasound signal is transmitted and the time when the ultrasound echo signal is received. In addition, the processor may generate an ultrasound image including temperature information by determining the temperature of the organ where the ultrasound echo signal is generated, from the amount of a change in the echo signal with respect to a reference echo signal. The processor 20 may include an array of a plurality of logic gates or a combination of a general microprocessor and a memory in which a program that may be executed by the microprocessor is stored. However, the present disclosure is not limited thereto, and the processor 20 may be implemented using different hardware.

In order to generate an ultrasound image including temperature information, the processor 20 may include a signal change amount calculation unit 210 that calculates the amount of a change in the echo signal with respect to the reference echo signal, a coefficient change amount calculation unit 220 that calculates the amount of a change in a back scattering coefficient of the organ and the amount of a change in an attenuation coefficient of the organ from the amount of the change in the echo signal, a temperature determination unit 230 that determines temperature of the place where the echo signal is generated, from the amount of the change in the back scattering coefficient of the organ, and an image generating unit 240 that generates an ultrasound image including temperature information.

The user input unit 50 receives user's input information. The user input unit 40 may include a control panel, a keyboard, a mouse, or the like. The display unit 30 displays the ultrasound image including the temperature information generated by the processor 20. The temperature information may be displayed as brightness, however, the present disclosure is not limited thereto.

For the convenience of explanation, an operation of determining a temperature of the organ by using the processor 20, which uses an intensity of a signal, will now be described below.

First, the transmission signal formation unit 110 may generate an ultrasound signal having an intensity $I_0$, and the ultrasound probe 120 may transmit the ultrasound signal to the organ.

Then, the ultrasound signal moves toward the organ that is at a distance **z** from the ultrasound probe 120, and then is scattered backwards. An intensity $I_s(z,T)$ of the ultrasound echo signal that is back-scattered and received by the ultrasound probe 120 may be expressed by Equation 1:

【Equation 1】

$$I_s(z,T) = \eta(z,T) I_0 \exp\left(-\int_0^{z-\Delta z} \alpha(z,T)dz\right)$$

where, $\eta(\textbf{z},\textbf{T})$ is a back scattering coefficient of the organ at a distance z, and $\alpha(\textbf{z},\textbf{T})$ is an attenuation coefficient of the organ at the distance z, and $-\Delta\textbf{z}$ is the size of a window regionat which the distance z is defined, and T is the temperature of the organ. The window region $\Delta z$ is a unit region to calculate an amount of a change in the echosignal

The back scattering coefficient $\eta(z,T)$ of the organ and the attenuation coefficient $\alpha(z,T)$ of the organ are changed according to the distance between the ultrasound probe 120 and the organ.

The storage unit 40 may include a reference echo signal database in which the intensity of the reference echo signal according to the distance between the ultrasound probe 120 and the organ at a reference temperature $T_R$ is stored. The reference echo signal may be stored in the reference echo signal database according to organs.

The following Equation 2 represents an intensity $I_s(z,T_R)$ of the reference echo signal that is back scattered after the ultrasound signal proceeds to the point z that is a region in which the ultrasound signal is distant from the ultrasound probe 120 by z at the reference temperature $T_R$. Here, the reference temperature $T_R$ may be temperature of the organ before the ultrasound signal is irradiated on the organ for treatment. That is, the intensity of the reference echo signal may correspond to an echo signal that is measured by ultrasound for diagnosis purposes before ultrasound for treatment is irradiated on the organ.

【Equation 2】

$$I_s(z,T_R) = \eta(z,T_R)I_0 \exp\left(-\int_0^{z-\Delta z} \alpha(z,T_R)dz\right)$$

.

The signal change amount calculation unit 210 calculates the amount of a change in the received echo signal with respect to the reference echo signal at the point z. The amount $CBE(z,T)$ of the change in the received echo signal may be expressed by Equation 3:

【Equation 3】

$$CBE(z,T) = \frac{I_s(z,T)}{I_s(z,T_R)}$$

$$= \frac{\eta(z,T)}{\eta(z,T_R)} \exp\left(-\int_0^{z-\Delta z} (\alpha(z,T) - \alpha(z,T_R))dz\right)$$

$$= \frac{\eta(z,T)}{\eta(z,T_R)} \exp\left(\int_0^{z-\Delta z} \Delta\alpha(\zeta,T)d\zeta\right)$$

.

As defined in Equation 3, the amount of the change in the echo signal may be twice the amount of a change in the back scattering coefficient of the organ and the amount of a change in the attenuation coefficient of the organ. In addition, the back scattering coefficient and the attenuation coefficient of the organ may be independent of each other.

When it is assumed that the attenuation coefficient of the organ is constant regardless of a temperature change, the amount of the change in the echo signal at the point z is the same as the amount of the change in the back scattering coefficient of the organ, as defined in the following Equation 4:

【Equation 4】

$$CBE'(z,T) = \frac{I_s(z,T)}{I_s(z,T_R)} = \frac{\eta(z,T)}{\eta(z,T_R)}$$

.

According to the empirical result when the attenuation coefficient of the organ is assumed to be constant regardless of temperature, the amount of the change in the back scattering coefficient of the organ may be defined by the following Equation 5:

【Equation 5】

$$\frac{\eta(T)}{\eta(T_R)} = \frac{\left(\frac{\rho_m c(T)_m^2 - \rho_s c(T)_s^2}{\rho_s c(T)_s^2}\right)^2 + \frac{1}{3}\left(\frac{3\rho_s - 3\rho_m}{2\rho_s + \rho_m}\right)^2}{\left(\frac{\rho_m c(T_R)_m^2 - \rho_s c(T_R)_s^2}{\rho_s c(T_R)_s^2}\right)^2 + \frac{1}{3}\left(\frac{3\rho_s - 3\rho_m}{2\rho_s + \rho_m}\right)^2}$$

where $\rho$ is a density, and $c(T)$ is the speed of ultrasound waves, and subscripts m and s denote an organ and a scattering body in the organ, respectively. The speed $c(T)$ of ultrasound waves may be indicated by a temperature function. Thus, the temperature of the organ may be determined from the amount of the change in the back scattering coefficient of the organ. Equation 5 theoretically represents the relationship between the amount of the change in the back scattering coefficient of the organ and the temperature of the organ, and thus, may be more precisely obtained through experiments, and a temperature that matches the amount of the change in the back scattering coefficient of the organ obtained through experiments may be stored and be used later.

The attenuation coefficient of the organ is changed according to the temperature change, as defined in Equation 3. According to the empirical result, the amount of the change in the attenuation coefficient $\Delta\alpha(\xi,T)$ of the organ at a point $\zeta$ (where $0 < \zeta < z - -\Delta z$) may be defined as in the following Equation 6:

【Equation 6】

$$\Delta\alpha(\zeta,T) = -kstd(\log(CBE(\zeta,T)))$$

where, $std(\log(CBE(\zeta, T))$ is a standard deviation with respect to the amount of the change in the echo signal at the point $\zeta$ at a former location, and k is a proportional constant. The value of k may be determined through experiments and depends on the type of the organ.

As defined in Equation 6, the amount of the change in the attenuation coefficient $\Delta\alpha(\zeta,T)$ of the organ may be proportional to the standard deviation with respect to the amount of the change in the echo signal at the point $\zeta$. That is, when there is no temperature change in the organ, the standard deviation with respect to the amount of the change in the echo signal is 0. Thus, the amount of the change in the attenuation coefficient of the organ according to a temperature change is 0. However, as the temperature change in the organ increases, the standard deviation with respect to the amount of the change in the echo signal increases. Thus, the amount of the change in the attenuation coefficient of the organ increases according to the standard deviation.

Thus, the coefficient change amount calculation unit 220 calculates the amount of the change in the attenuation coefficient of the organ from the standard deviation with respect to the amount of the change in the echo signal.

As described above, since the amount of the change in the attenuation coefficient of the organ is determined, based on the standard deviation with respect to the amount of the change in the echo signal, the amount of the change in the echo signal that is back scattered at the point z may be expressed by Equation 7:

【Equation 7】

$$CBE(z,T)$$

$$= \frac{\eta(z,T)}{\eta(z,T_R)}\exp\left(\int_0^{z-\Delta z}\Delta\alpha(\zeta,T)d\zeta\right)$$

$$= \frac{\eta(z,T)}{\eta(z,T_R)}\exp\left(-k\int_0^{z-\Delta z}std(\log(CBE(\zeta,T)))d\zeta\right)$$

.

Thus, the coefficient change amount calculation unit 220 may calculate the amount of the change in the back scattering coefficient of the organ at the point z from the standard deviation between the amount of the change in the echo signal that is back scattered at the point z and the amount of the change in the echo signal at the former point than the point z. The amount of the change in the back scattering efficient of the organ may be expressed by Equation 8:

【Equation 8】

$$\frac{\eta(z,T)}{\eta(z,T_R)}$$

$$= CBE(z,T)\exp\left(\int_0^{z-\Delta z} \Delta\alpha(\zeta,T)d\zeta\right)$$

$$= CBE(z,T)\exp\left(k\int_0^{z-\Delta z} std(\log(CBE(\zeta,T)))d\zeta\right).$$

The temperature determination unit 230 may determine the temperature of the organ at the point z from the amount of the change in the back scattering coefficient of the organ at the point z that is obtained by applying Equation 5 to the amount of the change in the back scattering coefficient of the organ that is calculated using Equation 8.

Whenever the temperature of the organ is determined from the amount of the change in the back scattering coefficient of the organ by using Equation 5, a load may be generated. Thus, a temperature database in which the amount of the change in the back scattering coefficient of the organ and the temperature of the organ are mapped to each other may be stored in the storage unit 40. Thus, the coefficient change amount calculation unit 220 calculates the amount of the change in the back scattering coefficient of the organ from the amount of the change in the echo signal, and the temperature determination unit 230 may determine the temperature of the organ by reading the temperature of the organ that is mapped to the amount of the change in the back scattering coefficient of the organ, from the temperature database.

The image generating unit 240 generates an ultrasound image including information regarding the temperature of each point of the organ. A method of generating an ultrasound image by using an ultrasound signal is known to one of ordinary skill in the art, and thus, a detailed description thereof will not be provided here. However, according to an example embodiment, the image generating unit 240 may generate the ultrasound image including the temperature information by adding the information regarding the temperature of each point of the organ to the aforementioned ultrasonic image. The temperature information may be expressed as brightness, however, the present disclosure is not limited thereto.

FIG. 4 is a flowchart illustrating an operation of determining a temperature of an organ from the amount of a change in an echo signal, according to an embodiment of the present disclosure.

First, the ultrasound data obtaining unit 10 irradiates ultrasound signal on the organ. The ultrasound data obtaining unit 10 receives an echo signal from the organ in operation S410. The echo signal is back scattered by the scattering body in the organ and is received by the ultrasound probe 120.

The signal change amount calculation unit 210 of the processor 20 calculates the amount of a change in the echo signal with respect to a reference echo signal in operation S420. The reference echo signal may be an echo signal that is received when the organ is at a reference temperature. The amount of a change in the echo signal may be represented as an intensity of the echo signal. The coefficient change amount calculation unit 220 of the processor 20 calculates the amount of a change in an attenuation coefficient of the organ from the amount of the change in the echo signal at the former point than the point of the organ in operation S430, and calculates the amount of a change in a back scattering coefficient of the organ from the amount of the change in the attenuation coefficient of the organ and the amount of the change in the echo signal in the organ in operation S440. Then, the temperature determination unit 230 determines temperature of the organ using the amount of the change in the back scattering coefficient of the organ in operation S450. Lastly, the image generating unit 240 generates an ultrasound image including information regarding the temperature of the organ in operation S460.

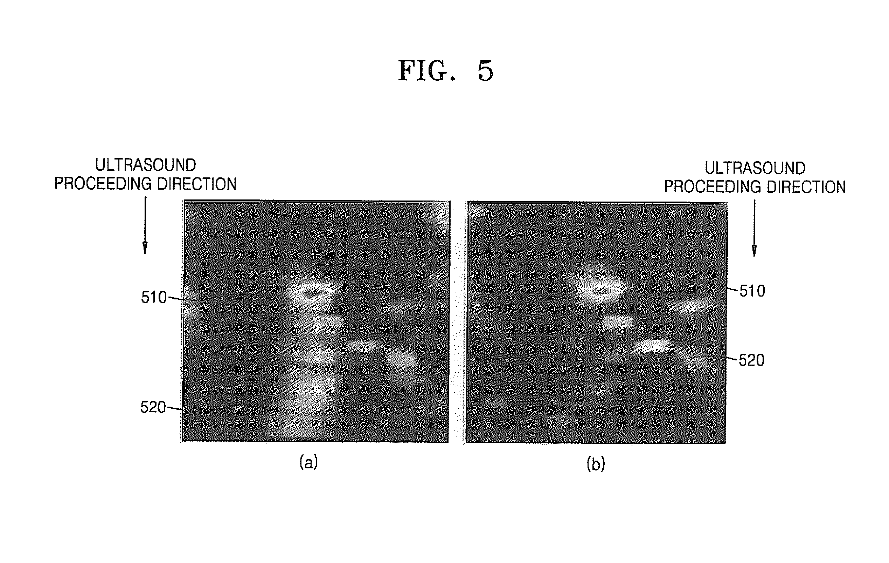

FIG. 5, part (a) and part (b) illustrate an ultrasound image including temperature information generated, according to the related art, and an ultrasound image including temperature information generated, according to the present disclosure, respectively. A temperature change occurs in a region 510. However, as illustrated in FIG. 5 part (a), since there is no compensation for a coefficient of the organ according to the temperature change in the related art, a false image appears in the ultrasound image in which a temperature change occurs even though there is no actual temperature change in a deeper region 520, other than the region 510 in which a change in the temperature of the organ with a tumor occurs.

However, when the coefficient of the organ is compensated for according to the temperature change, as illustrated in FIG. 5 part (b), the false image that appears in the deeper region 520 than the region 510 in which the temperature change occurs may be removed. Thus, it may be determined from the ultrasound image including the temperature information whether tumor necrosis occurred and whether a tissue is normal.

The embodiments can be implemented in computing hardware (computing apparatus) and/or software, such as (in a non-limiting example) any computer that can store, retrieve, process and/or output data and/or communicate with other computers. The results produced can be displayed on a display of the computing hardware. A program/software implementing the embodiments may be recorded on non-transitory computer-readable media comprising computer-readable recording media. Examples of the computer-readable recording media include a magnetic recording apparatus, an optical disk, a magneto-optical disk, and/or a semiconductor memory (for example, RAM, ROM, etc.). Examples of the magnetic recording apparatus include a hard disk device (HDD), a flexible disk (FD), and a magnetic tape (MT). Examples of the optical disk include a DVD (Digital Versatile Disc), a DVD-RAM, a CD-ROM (Compact Disc - Read Only Memory), and a CD-R (Recordable)/RW.

Further, according to an aspect of the embodiments, any combinations of the described features, functions and/or operations can be provided.

Moreover, the ultrasound system 1 may include at least one processor to execute at least one of the above-described units and methods.

As described above, according to the one or more of the above embodiments of the present description, the temperature of an organ is determined by using the amount of a change in an attenuation coefficient of the organ according to a temperature change so that tumor necrosis and safety of a normal tissue may be clearly checked.

While this disclosure has been particularly shown and described with reference to preferred embodiments thereof, it will be understood by those skilled in the art that various changes in form and details may be made therein without departing from the spirit and scope of the invention as defined by the appended claims. The preferred embodiments should be considered in descriptive sense only and not for purposes of limitation. Therefore, the scope of the invention is defined not by the detailed description of the invention but by the appended claims, and all differences within the scope will be construed as being included in the present invention.

**Claims**

1. A method of generating an ultrasound image, comprising:

   obtaining an echo signal from an ultrasound signal irradiated on an organ;
   calculating an amount of a change in the echo signal with respect to a reference echo signal;
   determining a temperature of a predetermined region of the organ from the amount of the change in the echo signal, using an amount of a change in an attenuation coefficient of the organ that changes according to a temperature change; and
   generating the ultrasound image comprising information about the determined temperature.

2. The method of claim 1, wherein the reference echo signal is an echo signal from an ultrasound signal irradiated on the organ when the organ is at a reference temperature.

3. The method of any one of the previous claims, wherein the amount of the change in the attenuation coefficient of the organ is determined from an amount of a change in a former echo signal that is an amount of a change in an echo signal at a former region different from the predetermined region of the organ in an ultrasound probe that irradiates ultrasound on the organ.

4. The method of claim 3, wherein the used amount of the change in the attenuation coefficient of the organ is proportional to a standard deviation with respect to the amount of the change in the former echo signal.

5. The method of claim 4, wherein, depending on the type of the organ, a predetermined degree of proportion with respect to the standard deviation between the amount of the change in the attenuation coefficient of the organ and the amount of the change in the former echo signal is used.

6. The method of any one of the previous claims, wherein the determining of the temperature of the organ comprises:

   calculating an amount of a change in a back scattering coefficient of the organ from the amount of the change in the attenuation coefficient of the organ and the amount of the change in the echo signal; and

determining the temperature of the organ, using the amount of change in back scattering coefficient.

7. The method of any one of the previous claims, wherein the temperature of the organ is determined by reading a temperature of the organ that is mapped to an amount of a change in a back scattering coefficient of the organ, from a temperature database in which the amount of the change in the back scattering coefficient of the organ and the temperature of the organ are mapped to each other.

8. A non-transitory computer readable recording medium having recorded thereon a program for executing the method of any one of the previous claims.

9. An ultrasound system, comprising:

a ultrasound data obtaining unit adapted to irradiate ultrasound on an organ, and to receive an echo signal from a predetermined region of the organ; and
a processor adapted to calculate an amount of a change in the echo signal with respect to a reference echo signal, and to determine a temperature of the predetermined region from the amount of the change in the echo signal, using an amount of a change in an attenuation coefficient of the organ that changes according to a temperature change.

10. The ultrasound system of claim13, wherein the reference echo signal is an echo signal of ultrasound irradiated on the organ, when the organ is at a reference temperature.

11. The ultrasound system of claim9 or 10, wherein the processor is adapted to determine the amount of the change in the attenuation coefficient of the organ from an amount of a change in a former echo signal that is an amount of a change in an echo signal at a former region different from the predetermined region in an ultrasound probe that irradiates ultrasound on the organ.

12. The ultrasound system of claim 11, wherein the amount of the change in the attenuation coefficient of the organ is proportional to a standard deviation with respect to the amount of the change in the former echo signal.

13. The ultrasound system of claim12, wherein a degree of proportion with respect to the standard deviation between the amount of the change in the attenuation coefficient of the organ and the amount of the change in the former echo signal depends on a type of the organ.

14. The ultrasound system of any one of the claims 9-13, wherein the processor is adapted to calculate an amount of a change in a back scattering coefficient of the organ from the amount of the change in the attenuation coefficient of the organ and the amount of the change in the echo signal and to determine the temperature of the organ from the back scattering coefficient.

15. The ultrasound system of any one of the claims19-14 further comprising a display unit adapted for displaying an ultrasound image comprising information regarding the temperature of the organ.

# FIG. 1

```
                                    40
                          ┌──────────────────┐
                          │   STORAGE UNIT   │
                          └──────────────────┘
          10                       ↕ 20                 30
┌──────────────────┐      ┌──────────────────┐   ┌──────────────────┐
│ ULTRASOUND DATA  │ ───→ │    PROCESSOR     │ → │   DISPLAY UNIT   │
│  OBTAINING UNIT  │      └──────────────────┘   └──────────────────┘
└──────────────────┘               ↑ 50
                          ┌──────────────────┐
                          │  USER INPUT UNIT │
                          └──────────────────┘
```

1

# FIG. 2

10

```
          110
┌──────────────────────┐
│ TRANSMISSION SIGNAL  │
│   FORMATION UNIT     │
└──────────────────────┘
          │ 120                    130                    140
          ↓
┌──────────────────────┐   ┌──────────────────┐   ┌──────────────────────┐
│   ULTRASOUND PROBE   │ → │   BEAM FORMER    │ → │  ULTRASOUND DATA     │
└──────────────────────┘   └──────────────────┘   │   FORMATION UNIT     │
                                                   └──────────────────────┘
```

# FIG. 3

20

210      220      230      240

| SIGNAL CHANGE AMOUNT CALCULATION UNIT | COEFFICIENT CHANGE AMOUNT CALCULATION UNIT | TEMPERATURE DETERMINATION UNIT | IMAGE GENERATING UNIT |

# FIG. 4

RECEIVE ECO SIGNAL OF ULTRASOUND —S410

CALCULATE AMOUNT OF CHANGE IN ECO SIGNAL —S420

CALCULATE AMOUNT OF CHANGE IN ATTENUATION COEFFICIENT OF ORGAN —S430

CALCULATE AMOUNT OF CHANGE IN BACK SCATTERING COEFFICIENT OF ORGAN —S440

DETERMINE TEMPERATURE OF ORGAN —S450

GENERATE ULTRASOUND IMAGE INCLUDING TEMPERATURE INFORMATION —S460

# FIG. 5

EP 2 559 376 A1

ULTRASOUND
PROCEEDING DIRECTION

510

520

(a)

ULTRASOUND
PROCEEDING DIRECTION

510

520

(b)

Europäisches
Patentamt

European
Patent Office

Office européen
des brevets

## EUROPEAN SEARCH REPORT

Application Number

EP 12 18 0879

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SUPRATIC CHAKRABOR1Y AND MADHUSUDAN ROY: "Noninvasive temperature imaging through ultrasonic attenuation", JOURNAL OF PURE AND APPLIED ULTRASONICS,, vol. 28, no. 2-4, 1 April 2006 (2006-04-01), pages 129-134, XP008157867, | 1,7-9,15 | INV. A61B5/01 A61B8/00 A61N7/02 G01K11/02 |
| Y | * abstract * * page 129, left-hand column, paragraph 1; figures 1,2a,3 * * page 130, left-hand column, paragraph 2 * * page 130, right-hand column, paragraph 4 - page 133, left-hand column, paragraph 3 * | 4,5,12, 13 | |
| X | US 4 807 633 A (FRY FRANCIS J [US]) 28 February 1989 (1989-02-28) * column 1, line 22 - line 30 * * column 2, line 19 - line 48 * * column 3, line 1 - column 6, line 62; claims 1-8; figures 1-5 * | 1-3,6, 8-11,14 | |
| X | US 4 754 760 A (FUKUKITA HIROSHI [JP] ET AL) 5 July 1988 (1988-07-05) | 1-3,8-11 | TECHNICAL FIELDS SEARCHED (IPC) A61B A61N G01K G01N |
| A | * column 4, line 45 - column 5, line 21; figure 2 * * column 9, line 2 - line 33 * | 15 | |
| X | US 6 623 430 B1 (SLAYTON MICHAEL H [US] ET AL) 23 September 2003 (2003-09-23) * column 4, line 60 - column 5, line 40 * * column 10, line 20 - column 12, line 40; figures 2-6 * | 1,8,9 | |

-/--

The present search report has been drawn up for all claims

| Place of search | Date of completion of the search | Examiner |
|---|---|---|
| The Hague | 12 November 2012 | Daoukou, Eleni |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

**EUROPEAN SEARCH REPORT**

Application Number

EP 12 18 0879

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 513 750 A (HEYMAN JOSEPH S [US] ET AL) 30 April 1985 (1985-04-30) <br> * column 2, line 46 - columns 3,30; figure 1 * <br> ----- | 1,8,9 | |
| Y | US 2011/087097 A1 (BEHAR BOAZ [IL]) 14 April 2011 (2011-04-14) <br> * paragraphs [0020], [0021], [0050], [0051], [0074], [0075], [0086] * <br> ----- | 4,5,12, 13 | |
| A | CHRISTAKIS A. DAMIANOU ET AL: "Dependence of ultrasonic attenuation and absorption in dog soft tissues on temperature and thermal dose", <br> THE JOURNAL OF THE ACOUSTICAL SOCIETY OF AMERICA, <br> vol. 102, no. 1, 1 July 1997 (1997-07-01), pages 628-634, XP055043913, <br> ISSN: 0001-4966, DOI: 10.1121/1.419737 <br> * page 631, right-hand column, paragraph 4 - page 632, left-hand column, paragraph 1; figures 4,6 * <br> ----- | 1-15 | **TECHNICAL FIELDS SEARCHED** (IPC) |

The present search report has been drawn up for all claims

| Place of search | Date of completion of the search | Examiner |
|---|---|---|
| The Hague | 12 November 2012 | Daoukou, Eleni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 12 18 0879

This annex lists the patent family members relating to the patent documents cited in the above-mentioned European search report.
The members are as contained in the European Patent Office EDP file on
The European Patent Office is in no way liable for these particulars which are merely given for the purpose of information.

12-11-2012

| Patent document cited in search report | | Publication date | Patent family member(s) | | Publication date |
|---|---|---|---|---|---|
| US 4807633 | A | 28-02-1989 | NONE | | |
| US 4754760 | A | 05-07-1988 | JP | 1841426 C | 12-05-1994 |
| | | | JP | 5070769 B | 05-10-1993 |
| | | | JP | 63122923 A | 26-05-1988 |
| | | | US | 4754760 A | 05-07-1988 |
| US 6623430 | B1 | 23-09-2003 | NONE | | |
| US 4513750 | A | 30-04-1985 | NONE | | |
| US 2011087097 | A1 | 14-04-2011 | EP | 2519157 A1 | 07-11-2012 |
| | | | EP | 2519159 A1 | 07-11-2012 |
| | | | US | 2011087097 A1 | 14-04-2011 |
| | | | WO | 2011080712 A1 | 07-07-2011 |
| | | | WO | 2011080713 A1 | 07-07-2011 |

EPO FORM P0459

For more details about this annex : see Official Journal of the European Patent Office, No. 12/82